# EUROPEAN PATENT SPECIFICATION

(11) **EP 4 162 503 B1**
(45) Date of publication and mention of the grant of the patent: **04.03.2026**
(21) Application number: 21728886.9
(22) Date of filing: 26.05.2021
(51) Int. Cl.: G16H 30/20, G16H 40/20, G16H 40/67, G16H 80/00

(54) **SYSTEM AND METHOD FOR DATA-DRIVEN SELECTION OF A REMOTE EXPERT FOR A MEDICAL IMAGING EXAMINATION**
SYSTEM UND VERFAHREN ZUR DATENGESTEUERTEN AUSWAHL EINES ENTFERNTEN EXPERTEN FÜR EINE MEDIZINISCHE BILDGEBUNGSUNTERSUCHUNG
SYSTÈME ET PROCÉDÉ DE SÉLECTION PILOTÉE PAR LES DONNÉES D'UN EXPERT À DISTANCE POUR UN EXAMEN D'IMAGERIE MÉDICALE

(30) Priority: 03.06.2020 US 202062704911 P
(43) Date of publication of application: 12.04.2023
(73) Proprietor: Koninklijke Philips N.V., 5656 AG Eindhoven (NL)
(72) Inventor: CHAMARTHI, Hareesh, 5656 AE Eindhoven (NL); STAROBINETS, Olga, 5656 AE Eindhoven (NL); DALAL, Sandeep, Madhukar, 5656 AE Eindhoven (NL); TELLIS, Ranjith, Naveen, 5656 AE Eindhoven (NL); QIAN, Yuechen, 5656 AE Eindhoven (NL)
(74) Representative: Philips Intellectual Property & Standards
(86) International application number: PCT/EP2021/063948
(87) International publication number: WO 2021/244906

(56) References cited:
- US-A1- 2012 166 546

## Description

The following relates generally to the imaging arts, remote imaging assistance arts, remote imaging examination monitoring arts, and related arts.

### BACKGROUND

Currently, diagnostic imaging is in high demand. As the world population ages, the demand for quick, safe, high quality imaging may only continue to grow, putting further pressure on imaging centers and their staff. In order to perform imaging examinations on patients quickly and safely, maintaining high throughput and quality standards, an imaging provider has to establish an efficient workflow, safeguarding it from disruptions.

Some common workflow disruptions include lack of proper guidance for newly appointed technicians, technicians' lack of experience on imaging machines from various vendors with different control interfaces and features, lack of knowledge of imaging protocols, and so forth. These are examples of some common events that, if encountered at the time of the scan, may adversely impact the current imaging examination, and delay the following exams, potentially disrupting the entire workday. With proper preparation, by providing over the shoulder guidance to the technicians, many of these disruptions could be mitigated or avoided entirely.

The following discloses certain improvements to overcome these problems and others.

US 2012/166546 A1 discloses selecting a specialist from a list of available mobile device users for consultation regarding the selected study image.

### SUMMARY

In one aspect, a non-transitory computer readable medium stores instructions executable by at least one electronic processor to perform a method of connecting a local medical imaging device operator with a remote medical imaging expert during an imaging examination performed using a medical imaging device. The method includes: determining characteristics for available remote medical imaging experts who are available to assist the local operator; matching one or more of the available remote medical imaging experts based on the determined characteristics for the remote medical imaging experts with characteristics of the imaging examination; and providing a user interface (UI) to at least one display device operable by the local operator and the remote medical imaging expert, the UI displaying a list of the matched available remote medical imaging experts and via which the local medical imaging device operator can select a matched available medical imaging expert from the displayed list.

In another aspect, an apparatus for connecting a local medical imaging device operator during an imaging examination performed using a medical imaging device includes: a screen-sharing device for sharing a screen of a controller of the medical imaging device. A telephonic or video communication link is operatively connected with an electronic network for providing telephonic or video communication with a remote medical imaging expert of a set of remote medical imaging experts. A database stores determining characteristics for the remote medical imaging experts of the set of remote medical imaging experts in which the characteristics including at least one of an experience level with a modality of the imaging examination; an experience level with an anatomy of a patient to be imaged during the imaging examination; an experience level of working with the local operator; and an experience level with a type of problem occurring in the imaging examination. At least one electronic processor is programmed to: retrieve, from the database, characteristics for one or more remote medical imaging experts of the set of remote medical imaging experts who are available to assist the local operator in the imaging examination; rank the available remote medical imaging experts based on matching the characteristics of the available remote medical imaging experts with characteristics of the imaging examination; and provide a UI displaying a list of the ranked available remote medical imaging experts, enabling the local operator to select one of the listed available remote medical imaging experts, and establishing an assistance session between the local operator and the selected remote medical imaging expert via the screen-sharing device and the telephonic or video communication link.

In another aspect, a method of connecting a remote medical imaging expert to a local operator to provide assistance during an imaging examination includes: retrieving, from a database, characteristics for one or more remote medical imaging experts available to assist the local operator in an imaging examination; ranking the available remote medical imaging experts with a score indicative of the characteristics of the available remote medical imaging experts with the characteristics of the imaging examination; providing a UI displaying a list of the ranked available remote medical imaging experts; receiving a user input, via at least one user input device, indicative of a selection of one of the listed remote medical imaging experts; collecting data related to an effectiveness of assistance provided by the selected remote medical imaging expert and the local operator; determining one or more quality metrics related to the collected data; and updating the information stored in the database for the selected remote medical imaging expert with the calculated one or more quality metrics.

One advantage resides in providing a remote expert or radiologist assisting a technician in conducting a medical imaging examination with situational awareness of local imaging examination(s) which facilitates providing effective assistance to one or more local operators at different facilities.

Another advantage resides in providing a remote expert or radiologist to assist a technician in conducting a medical imaging examination, in which the remote expert or radiologist is well matched to the imaging modality and imaging examination.

Another advantage resides in preselecting a remote expert or radiologist to assist a technician in conducting an ongoing medical imaging examination, in which the remote expert or radiologist is well matched to the imaging modality and imaging examination, and in which the preselected remote expert or radiologist is prepared to quickly respond to any automatically detected error condition in the ongoing imaging examination.

Another advantage resides in providing a remote expert with information about already-performed steps in a workflow in order to provide assistance for subsequent steps in the workflow.

Another advantage resides in matching a most-suited remote expert for providing assistance to one or more local operators based on an experience level of the remote expert and features of the imaging examination performed by the one or more local operators.

Another advantage resides in providing a remote operator or radiologist with status information on a medical imaging examination using a standard display format that is independent of the controller display of the medical imaging device performing the medical imaging examination.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.
FIGURE 1 diagrammatically shows an illustrative apparatus for providing remote assistance in accordance with the present disclosure.
FIGURE 2 diagrammatically shows modules implemented by the apparatus of FIGURE 1.
FIGURE 3 shows an example of an output generated by the apparatus of FIGURE 1.
FIGURE 4 shows an example flow chart of operations suitably performed by the apparatus of FIGURE 1.

### DETAILED DESCRIPTION

The following related to Radiology Operations Command Center (ROCC) systems and methods that provide remote expert or "supertech" assistance to a local technician performing an imaging examination. There is value in providing rapid identification of a well-qualified supertech for assisting in a given imaging examination. Delays in providing the supertech can adversely impact imaging laboratory workflow. Moreover, in some disclosed embodiments, it is contemplated to provide supertech assistance in response to certain automatically detected errors or issues in the imaging examination, which again calls for the supertech to be immediately available upon detection of such an error.

In some embodiments disclosed herein, a supertech matching system matches the best available supertech with the local technician and/or with the current imaging examination. To this end, the system tracks available supertechs. A database stores information on each supertech relating to his/her expertise in various imaging modalities, imaged anatomies, and so forth. For matching to a specific local technician, the database stores similar information for the local technicians. For matching to a specific imaging procedure, information on the imaging procedure is stored. The database may have an ancillary information mining system for obtaining the information on the current imaging examination directly from the imaging scanner controller, or from the Radiology Information System (RIS) or other examination scheduling system.

As the database may store a wide range of features for characterizing the supertechs, local techs, and/or imaging examinations, a feature selection or reduction process may optionally be run for a given matching situation. For example, this may be implemented as a Principal Component Analysis (PCA) to generate highly discriminative features.

Based on the (optionally reduced) set of features, the available supertechs are matched to the local technician and/or to the imaging examination. In one matching approach, a clustering algorithm or other machine learning (ML) component groups the available supertechs by experience in various modalities (e.g., into tech levels 1-5 for a given modality and anatomy) and ranks the available supertechs based on these groupings. In another approach, a (non-machine learning) scoring system is employed to score how well each available supertech matches the local technician and/or examination, and the supertechs are ranked by score.

A user interface (UI) is provided via which the highest-matching supertech is presented to the local tech. In one approach, a list of the top-N highest matching supertechs are provided in a selection dialog, the local tech selects a supertech from the top-N selection dialog of the top N closest matching supertechs, and more detailed information about the selected supertech is displayed in a presentation window of the UI. If the local technician is satisfied with the selected supertech then a "connect" button or the like is selected to initiate an ROCC session with the selected supertech. In a variant of this approach, the UI may be presented to a third-party such as an ROCC administrator who selects the supertech and initiates the ROCC session.

In another variant embodiment, the UI is instead presented to the supertech. This approach may be appropriate in the case of an ROCC session triggered by an automatically detected error condition in an ongoing imaging examination. In this case, the UI would pop up to the highest-ranked supertech, with the displayed information being for the imaging examination in which the error occurred (and possibly also information on the local technician conducting that examination). This serves as an invitation which the supertech can then accept by activating a "connect" button or the like to initiate the ROCC session. Alternatively, the supertech can decline the invitation using a suitable UI dialog selector, in which case the system presents the invitation to the next-highest qualified supertech.

Optionally, the system can collect quality metrics, such as collecting data on whether a given matched supertech was able to effectively assist in the imaging examination. This collected data can be used by a human maintainer who may fine-tune the database features, machine learning component(s) or so forth to optimize performance of the system. In a variant approach, the system can employ adaptive learning such as pattern matching to adaptively adjust the machine learning component(s) to maximize metrics such as the effectiveness of the assistance provided by matched supertechs.

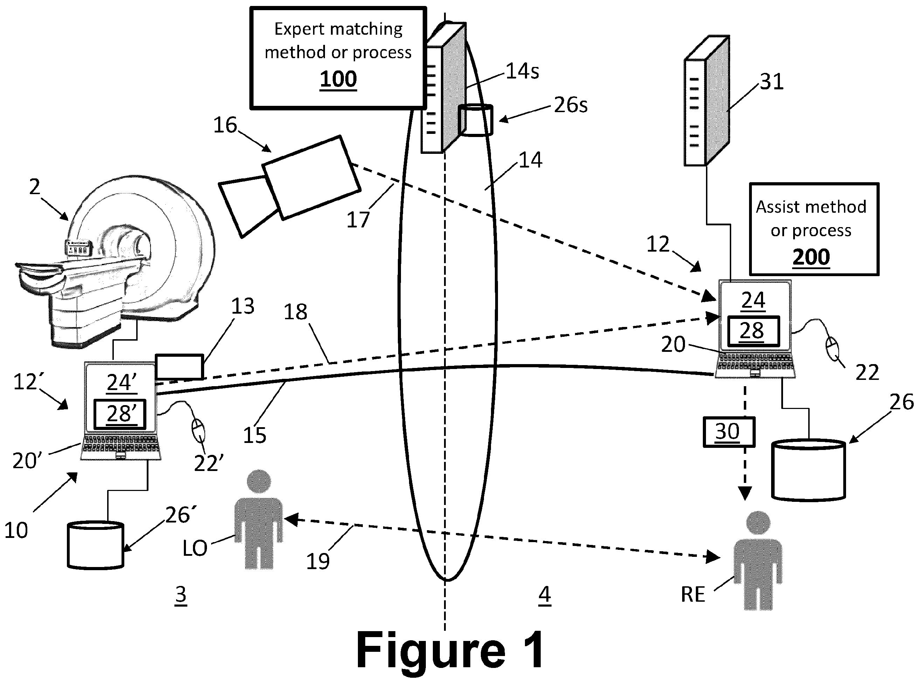

With reference to FIGURE 1, an apparatus for providing assistance from a remote medical imaging expert **RE** (or supertech) to a local technician operator **LO** is shown. As shown in FIGURE 1, the local operator **LO,** who operates a medical imaging device (also referred to as an image acquisition device, imaging device, and so forth) **2,** is located in a medical imaging device bay **3,** and the remote operator **RE** is disposed in a remote service location or center **4.** It should be noted that the "remote operator" **RE** may not necessarily directly operate the medical imaging device **2,** but rather provides assistance to the local operator **LO** in the form of advice, guidance, instructions, or the like. The remote location **4** can be a remote service center, a radiologist's office, a radiology department, and so forth. The remote location **4** may be in the same building as the medical imaging device bay **3** (this may, for example, in the case of a "remote operator" **RE** who is a radiologist tasked with peri-examination image review), but more typically the remote service center **4** and the medical imaging device bay **3** are in different buildings, and indeed may be located in different cities, different countries, and/or different continents. In general, the remote location **4** is remote from the imaging device bay **3** in the sense that the remote operator **RE** cannot directly visually observe the imaging device **2** in the imaging device bay **3** (hence optionally providing a video feed as described further herein).

The image acquisition device **2** can be a Magnetic Resonance (MR) image acquisition device, a Computed Tomography (CT) image acquisition device; a positron emission tomography (PET) image acquisition device; a single photon emission computed tomography (SPECT) image acquisition device; an X-ray image acquisition device; an ultrasound (US) image acquisition device; or a medical imaging device of another modality. The imaging device **2** may also be a hybrid imaging device such as a PET/CT or SPECT/CT imaging system. While a single image acquisition device **2** is shown by way of illustration in FIGURE 1, more typically a medical imaging laboratory will have multiple image acquisition devices, which may be of the same and/or different imaging modalities. For example, if a hospital performs many CT imaging examinations and relatively fewer MRI examinations and still fewer PET examinations, then the hospital's imaging laboratory (sometimes called the "radiology lab" or some other similar nomenclature) may have three CT scanners, two MRI scanners, and only a single PET scanner. This is merely an example. Moreover, the remote service center **4** may provide service to multiple hospitals. The local operator controls the medical imaging device **2** via an imaging device controller **10.** The remote operator is stationed at a remote workstation **12** (or, more generally, an electronic controller **12**).

As used herein, the term "medical imaging device bay" (and variants thereof) refer to a room containing the medical imaging device **2** and also any adjacent control room containing the medical imaging device controller **10** for controlling the medical imaging device. For example, in reference to an MRI device, the medical imaging device bay **3** can include the radiofrequency (RF) shielded room containing the MRI device **2,** as well as an adjacent control room housing the medical imaging device controller **10,** as understood in the art of MRI devices and procedures. On the other hand, for other imaging modalities such as CT, the imaging device controller **10** may be located in the same room as the imaging device **2,** so that there is no adjacent control room and the medical bay **3** is only the room containing the medical imaging device **2.** In addition, while FIGURE 1 shows a single medical imaging device bay **3,** it will be appreciated that the remote service center **4** (and more particularly the remote workstation **12**) is in communication with multiple medical bays via a communication link **14,** which typically comprises the Internet augmented by local area networks at the remote operator **RE** and local operator **LO** ends for electronic data communications.

As diagrammatically shown in FIGURE 1, in some embodiments, a camera **16** (e.g., a video camera) is arranged to acquire a video stream **17** of a portion of the medical imaging device bay **3** that includes at least the area of the imaging device **2** where the local operator **LO** interacts with the patient, and optionally may further include the imaging device controller **10.** The video stream **17** is sent to the remote workstation **12** via the communication link **14,** e.g. as a streaming video feed received via a secure Internet link.

In other embodiments, the live video feed **17** is, in the illustrative embodiment, provided by a video cable splitter **15** (e.g., a DVI splitter, a HDMI splitter, and so forth). In other embodiments, the live video feed **17** may be provided by a video cable connecting an auxiliary video output (e.g. aux vid out) port of the imaging device controller **10** to the remote workstation **12** of the operated by the remote expert **RE.**

Additionally or alternatively, a screen mirroring data stream **18** is generated by a screen sharing device **13,** and is sent from the imaging device controller **10** to the remote workstation **12.** The communication link **14** also provides a natural language communication pathway **19** for verbal and/or textual communication between the local operator and the remote operator. For example, the natural language communication link **19** may be a Voice-Over-Internet-Protocol (VOIP) telephonic connection, an online video chat link, a computerized instant messaging service, or so forth. Alternatively, the natural language communication pathway **19** may be provided by a dedicated communication link that is separate from the communication link **14** providing the data communications **17, 18,** e.g. the natural language communication pathway **19** may be provided via a landline telephone.

FIGURE 1 also shows, in the remote service center **4** including the remote workstation **12,** such as an electronic processing device, a workstation computer, or more generally a computer, which is operatively connected to receive and present the video **17** of the medical imaging device bay **3** from the camera **16** and to present the screen mirroring data stream **18** as a mirrored screen. Additionally or alternatively, the remote workstation **12** can be embodied as a server computer or a plurality of server computers, e.g. interconnected to form a server cluster, cloud computing resource, or so forth. The workstation **12** includes typical components, such as an electronic processor **20** (e.g., a microprocessor), at least one user input device (e.g., a mouse, a keyboard, a trackball, and/or the like) **22,** and at least one display device **24** (e.g. an LCD display, plasma display, cathode ray tube display, and/or so forth). In some embodiments, the display device **24** can be a separate component from the workstation **12.** The display device **24** may also comprise two or more display devices, e.g. one display presenting the video **17** and the other display presenting the shared screen of the imaging device controller **10** generated from the screen mirroring data stream **18.** Alternatively, the video and the shared screen may be presented on a single display in respective windows. The electronic processor **20** is operatively connected with a one or more non-transitory storage media **26.** The non-transitory storage media **26** may, by way of non-limiting illustrative example, include one or more of a magnetic disk, RAID, or other magnetic storage medium; a solid state drive, flash drive, electronically erasable read-only memory (EEROM) or other electronic memory; an optical disk or other optical storage; various combinations thereof; or so forth; and may be for example a network storage, an internal hard drive of the workstation **12,** various combinations thereof, or so forth. It is to be understood that any reference to a non-transitory medium or media **26** herein is to be broadly construed as encompassing a single medium or multiple media of the same or different types. Likewise, the electronic processor **20** may be embodied as a single electronic processor or as two or more electronic processors. The non-transitory storage media **26** stores instructions executable by the at least one electronic processor **20.** The instructions include instructions to generate a graphical user interface (GUI) **28** for display on the remote operator display device **24.**

The medical imaging device controller **10** in the medical imaging device bay **3** also includes similar components as the remote workstation **12** disposed in the remote service center **4.** Except as otherwise indicated herein, features of the medical imaging device controller **10,** which includes a local workstation **12',** disposed in the medical imaging device bay **3** similar to those of the remote workstation **12** disposed in the remote service center **4** have a common reference number followed by a "prime" symbol, and the description of the components of the medical imaging device controller **10** will not be repeated. In particular, the medical imaging device controller **10** is configured to display a GUI **28'** on a display device or controller display **24'** that presents information pertaining to the control of the medical imaging device **2,** such as configuration displays for adjusting configuration settings an alert **30** perceptible at the remote location when the status information on the medical imaging examination satisfies an alert criterion of the imaging device **2,** imaging acquisition monitoring information, presentation of acquired medical images, and so forth. It will be appreciated that the screen mirroring data stream **18** carries the content presented on the display device **24'** of the medical imaging device controller **10.** The communication link **14** allows for screen sharing between the display device **24** in the remote service center **4** and the display device **24'** in the medical imaging device bay **3.** The GUI **28'** includes one or more dialog screens, including, for example, an examination/scan selection dialog screen, a scan settings dialog screen, an acquisition monitoring dialog screen, among others. The GUI **28'** can be included in the video feed **17** or the mirroring data stream **17'** and displayed on the remote workstation display **24** at the remote location **4.**

FIGURE 1 also shows the remote workstation **12** in communication with a database **31** storing patient information (e.g., an electronic health record (EHR) database, an electronic medical record (EMR) database, a Radiology Information System (RIS) database, and so forth).

FIGURE 1 shows an illustrative local operator **LO,** and an illustrative remote expert **RE** (i.e. expert, e.g. supertech). However, in a Radiology Operations Command Center (ROCC) as contemplated herein, the ROCC provides a staff of supertechs who are available to assist a local operator **LO** at different hospitals, radiology labs, or the like. The ROCC may be housed in a single physical location, or may be geographically distributed. For example, in one contemplated implementation, the remote operators **RO** are recruited from across the United States and/or internationally in order to provide a staff of supertechs with a wide range of expertise in various imaging modalities and in various imaging procedures targeting various imaged anatomies. In view of this multiplicity of local operators **LO** and multiplicity of remote operators **RO,** the disclosed communication link **14** includes a server computer **14s** (or a cluster of servers, cloud computing resource comprising servers, or so forth) which is programmed to establish connections between selected local operator **LO**/remote expert **RE** pairs. For example, if the server computer **14s** is Internet-based, then connecting a specific selected local operator **LO**/remote expert **RE** pair can be done using Internet Protocol (IP) addresses of the various components **16, 10, 12,** the telephonic or video terminals of the natural language communication pathway **19,** et cetera. The server computer **14s** is operatively connected with a one or more non-transitory storage media **26s.** The non-transitory storage media **26s** may, by way of non-limiting illustrative example, include one or more of a magnetic disk, RAID, or other magnetic storage medium; a solid state drive, flash drive, electronically erasable read-only memory (EEROM) or other electronic memory; an optical disk or other optical storage; various combinations thereof; or so forth; and may be for example a network storage, an internal hard drive of the server computer **14s,** various combinations thereof, or so forth. It is to be understood that any reference to a non-transitory medium or media **26s** herein is to be broadly construed as encompassing a single medium or multiple media of the same or different types. Likewise, the server computer **14s** may be embodied as a single electronic processor or as two or more electronic processors. The non-transitory storage media **26s** stores instructions executable by the server computer **14s.** In addition, the non-transitory computer readable medium **26s** (or another database) stores data related to a set of remote experts **RE** and/or a set of local operators **LO.** The remote expert data can include, for example, skill set data, work experience data, data related to ability to work on multi-vendor modalities, data related to experience with the local operator **LO** and so forth.

Furthermore, as disclosed herein the server **14s** performs an expert matching method or process **100** that matches an available well-qualified remote expert **RE** with a given local operator **LO.**

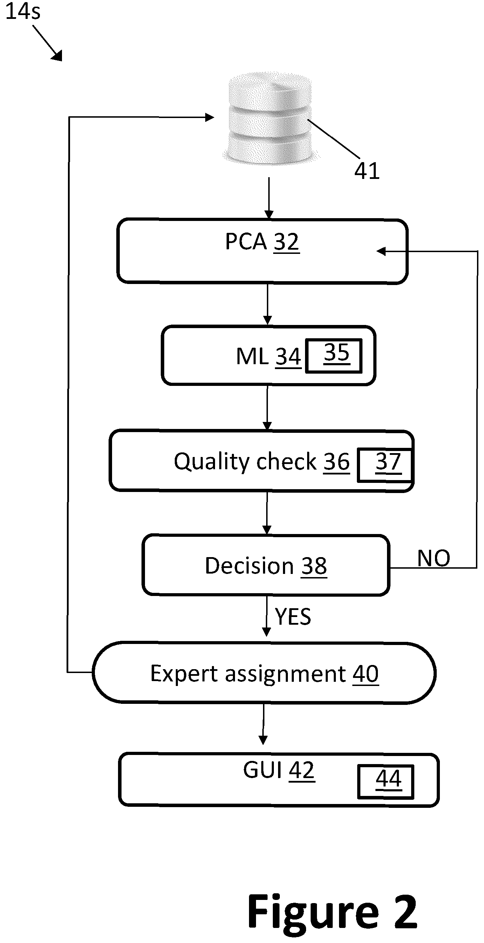

Referring now to FIGURE 2, and with continuing reference to FIGURE 1, in one embodiment of the expert matching method or process **100,** the server **14s** is programmed with several components to provide assistance to the remote expert **RE.** A principal component analysis model **32** is configured to analyze the remote expert data stored in a database **41** (which could be the non-transitory computer readable medium **26s**) and identify features to perform a medical imaging examination, such as a modality of the medical imaging device **2,** a vendor of the medical imaging device, a type of protocol to be used in the medical imaging examination, anatomy to be imaged, condition of the patient to be imaged, prior work experience of the local operator **LO** in handling relevant cases, prior work experience of the remote expert **RE** in handling relevant cases an availability of the remote experts, and so forth. In particular, the availability of each remote expert **RE** is retrieved. In some examples, the principal component analysis model **32** can be a machine-learning (ML) model.

The principal component analysis model **32** is configured to categorize the remote experts **RE** into skill levels on a scale of level 1-5. A "level 1 expert" can have around 0-2 years of experience be well-skilled in patient care and safety, and will need support and guidance in perfecting basics in obtaining images (e.g., from a more experienced super-tech). A "level 2 expert" can have around 2-3 years of experience, be well-skilled in patient care and safety, able to produce quality images, and will need to solidify knowledge to get confidence making independent decisions and exposure to new, more challenging cases. A "level 3 expert" has at least 3-5 years of experience, and has a strong ability in obtaining standard images. A "level 4 expert" has 5 or more years of experience, has a strong knowledge on advance imaging, can advise on examination cards, can anticipate most imaging protocols, and should be able to maintain and up-to-date relationship with other technicians on preferences. A "level 5 expert" has at least 10 years of experience, has strong knowledge on advance medical imaging, can set exam cards, can anticipate all imaging protocols, should be able to support on retaining all the knowledge to help local operators **LO,** and should be able to maintain up-to-date relationship with technologists on preferences.

Data characterizing the remote experts **RE** can be provided, as input to the principal component analysis model **32.** To do so, the remote experts **RE** can for example fill out questionnaires when joining the ROCC to provide information on their experience with different modalities, imaged anatomies, etc. In one embodiment, the principal component analysis model **32** includes, for example, five features for use in the model, where the features are calculated based on based on relevant work experience of the remote experts, and corresponding medical imaging examination complexity conditions. For example, one set of features could be obtained from a work experience of super-tech (labelled as ST-WE) combined with a complexity of the medical imaging examination to produce a binarized feature. If the first quartile, median, and third quartiles of the ST-WE and relativity to exam complexity (REC) or support required to perform the scan, are 10, 20, and 30, the following four features can be defined: "Is the ST-WE_REC value less than 10?" "Is the ST-WE_REC value greater than or equal to 10?"; "Is the ST-WE_REC value greater than or equal to 20?" and "Is the ST-WE_REC value greater than or equal to 30?". Other binarized features can be similarly obtained, such as ST-WE_ScannerType, ST-WE _Vendor, and so on. Other constraints to the principal component analysis model **32** can include constraints on accuracies such as an area under the curve (AUC) value.

A ML module **34** is configured to group all of the remote experts **RE** of the set of remote experts into various categories based on, for example their experience in working with different imaging modalities, patient condition, medical imaging examination complexity, modality, modality vendor, local operator **LO** experience, etc. One or more ML models 35 can be generated and used to predict a set of variables relevant for the medical imaging examination. The models **35,** along with the principal component analysis model **32,** and the remote expert **RE** data stored in the non-transitory computer readable medium **26** are used to find an "best-fit" remote expert to assist the local operator **LO** in the medical imaging examination. For example, the models **35** can be used to prioritize the remote experts **RE** into the following categories: exact match, relevant experience, exact examination experience but no modality experience, modality experience but no examination experience, and no modality or examination experience.

A quality metric check module **36** is configured simulate remote expert **RE** - local operator **LO** pairings make future pairing predictions, and also monitor quality metric values **37** such as the AUC values from the principal component analysis model **32** and the models **35.**

A decision-making module **38** is configured to determine whether results from the ML module **34** and the quality metric check module **36** meet a predetermined satisfactory threshold. If the threshold is not met, then the remote expert **RE** can change the input constraints to the principal component analysis model **32** and re-obtain a new set of remote experts based on the new input constraints, along with a new set of quality metric values **37.**

If the threshold is met, then an assignment module **40** is configured to identify the best remote expert **RE** (or a ranked top-N list of N most highly ranked experts **RE**) for the medical imaging examination (e.g., selecting accurate sequences for imaging and obtain high-quality images successfully) and match the best remote expert with the local operator **LO.** If a match is made, then the generated models **35** and quality metric values **37** can be stored in the database **41** (or, alternatively, in the non-transitory computer readable medium **26**) for use in future matchings.

A GUI output module **42** is configured to output a list **44** on the display device **24** of the remote workstation **12** (via the GUI **28**) and/or the display device **24'** of the medical imaging device controller **10** (via the GUI **28**') with the best available remote experts **RE,** along with one or more corresponding quality metric values **37** (e.g., confidence, values, AUC values, and so forth). The list **44** can include only a set number of best available remote experts **RE,** such as the three best available experts and the corresponding quality metric values **37.**

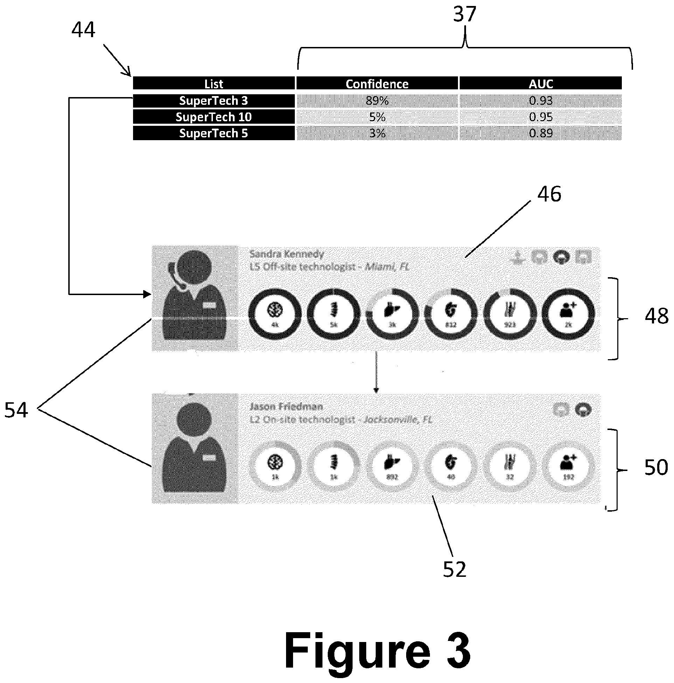

FIGURE 3 shows an example of a list **44.** As shown in FIGURE 3, the list **44** includes three best available remote experts **RE,** along with the corresponding quality metric values 37 including confidence values and AUC values. Upon a selection of one of the listed remote experts **RE** via the at least one user input device **22, 22',** a drop-down menu **46** listing a set of experience metrics **48** of the remote expert **RE** can be shown. As shown in FIGURE 3, the set of experience metrics **48** can include a brain imaging experience metric, a spine imaging experience metric, a liver imaging experience metric, a heart imaging experience metric, a knee imaging experience metric, and a whole-body imaging experience metric. These metrics **48** are compared to corresponding experience metrics **50** shown on a drop-down menu **52** of a local operator **LO.** From this, the best available remote expert **RE** can be selected to assist the local operator **LO.** In addition, the list **44** can also include a communication button **54** selectable by the remote expert **RE** or the local operator **LO** to establish the natural language communication pathway **19** via the communication link **14** between the two parties.

Upon selection of a remote expert **RE** to provide assistance to a given local operator **LO,** the communication link **14** connects the local operator **LO**/selected remote expert **RE.** The remote workstation **12** of the selected remote expert **RE,** and/or the medical imaging device controller **10** being run by the local operator **LO,** is configured to perform a method or process **200** for providing assistance from the remote expert **RE** to the local operator **LO.** For brevity, the method **200** will be described as being performed by the remote workstation **12.** The non-transitory storage medium **26** stores instructions which are readable and executable by the at least one electronic processor **20** (of the workstation **12,** as shown, and/or the electronic processor or processors of a server or servers on a local area network or the Internet) to perform disclosed operations including performing the method or process **200.**

A suitable implementation of the assistance method or process **200** is as follows. The method **200** is performed over the course of (at least a portion of) a medical imaging examination performed using the medical imaging device **2,** and the local expert **RE** is one selected via the matching method **100.** As used herein, the term "duration of a medical imaging examination" (or variants thereof) refers to a time period of a medical imaging examination that includes (i) an actual image acquisition time, (ii) imaging follow-on processing time, and (iii) up to a time of patient release. To perform the method **200,** the workstation **12** in the remote location **4** is programmed to receive at least one of: (i) the video **17** from the video camera **16** of the medical imaging device **2** located in the medical imaging device bay **3;** and/or (ii) the screen sharing **18** from the screen sharing device **19;** and/or (iii) the video **17** tapped by the video cable splitter **15.** The video feed **17** and/or the screen sharing **18** can be displayed at the remote workstation display **24,** typically in separate windows of the GUI **28.** The video feed **17** and/or the screen sharing **18** can be screen-scraped to determine information related to the medical imaging examination (e.g., modality, vendor, anatomy to be imaged, cause of issue to be resolved, and so forth). In particular, the GUI **28** presented on the display **24** of the remote workstation **12** preferably includes a window presenting the video **17,** and a window presenting the mirrored screen of the medical imaging device controller **10** constructed from the screen mirroring data stream **18,** and status information on the medical imaging examination that is maintained at least in part using the screen-scraped information. This allows the remote operator **RE** to be aware of the content of the display of the medical imaging device controller **10** (via the shared screen) and also to be aware of the physical situation, e.g. position of the patient in the medical imaging device **2** (via the video **17**), and to additionally be aware of the status of the imaging examination as summarized by the status information. During an imaging procedure, the natural language communication pathway **19** is suitably used to allow the local operator **LO** and the remote operator **RE** to discuss the procedure and in particular to allow the remote operator to provide advice to the local operator.

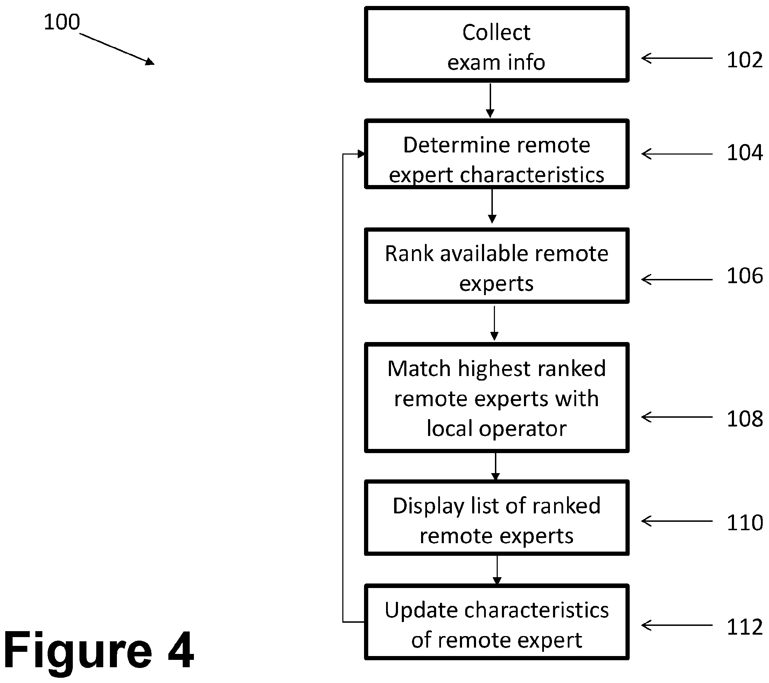

With reference to FIGURE 4, and with continuing reference to FIGURES 1-3, an illustrative embodiment of the expert matching method **100** is diagrammatically shown as a flowchart. At an operation **102,** information on the medical imaging examination to be performed by the local operator **LO** is collected. In one approach, the information is communicated to the server **14s** by an imaging laboratory scheduling system that scheduled the imaging examination. In another contemplated approach, the local operator **LO** fills out an electronic expert assistance request form pushed to the local operator **LO** by the server **14s** (e.g. as a webpage), and the form asks for relevant information about the imaging examination (e.g. modality, vendor, anatomy to be imaged, cause of issue to be resolved, and so forth). In yet another contemplated approach, the video feed **17** and/or the screen sharing **18** is captured at the server **14s** and is screen-scraped to determine information related to the medical imaging examination (e.g., modality, vendor, anatomy to be imaged, cause of issue to be resolved, and so forth). In some examples, the information collected during the operation **102** can include availability of different remote experts **RE.**

At an operation **104,** characteristics for available remote medical imaging experts **RE** who are available to assist the local operator **LO** are determined. To do so, the remote expert data stored in the non-transitory computer readable medium **26** can be retrieved and input to the principal component analysis model **32.** A feature selection process, such as a Principal Component Analysis (PCA) can be performed on the retrieved data and the screen-scraped information from the video **17** and the screen sharing **18** to generate the principal component analysis model **32** and determine the characteristics of the available remote experts **RE.** The characteristics of the available remote experts **RE** can include at least one of: an experience level with an imaging modality of the imaging examination; an experience level with an anatomy of a patient to be imaged during the imaging examination; an experience level of working with the local operator **LO;** an experience level with a type of problem occurring in the imaging examination, and so forth. In some examples, the availability data collected at the operation **102** can be combined with scheduling information of the imaging examination, to not only determine which remote experts **RE** are available, but the duration of the availability. This allows comparison of the expected duration of the imaging examination with the duration of availability of remote experts, so as to avoid a situation in which the remote expert becomes unavailable before the imaging examination (including follow-on processing time, up to patient release) is completed. This reduces likelihood that the patient will not need to re-visit. A list of "soon-to-be available remote experts **RE** can also be included.

At an operation **106,** the available remote experts **RE** are ranked. To do so, a ML operation is applied to the principal component analysis model **32** to rank the available remote experts **RE.** This is performed using the ML module **34** and the generated models **35.** In addition, the quality metric values **37** are generated using the quality metric check module **38,** which are also used to rank the available remote experts **RE.** The quality metric values **37** are used as scores to rank the remote experts **RE.** The decision making module **38** then ranks the available remote experts **RE** based on the quality metrics **37** and the results of the models **35** generated by the ML module **34.**

At an operation **108,** one or more of the available remote experts **RE** are matched with the local operator **LO** performing the medical imaging examination. This is performed using the assignment module **40.** The best remote expert **RE** for the medical imaging examination (e.g., selecting accurate sequences for imaging and obtain high-quality images successfully) and match the best remote expert with the local operator **LO.**

At an operation **110,** the list **44** of ranked available remote experts **RE** is displayed via the GUI **28** on the display device **24** of the remote workstation **12** and/or via the GUI **28'** on the display device **24'** of the medical imaging device controller **10.** Referring back to FIGURE 2, the list **44** can include the quality metrics **37** as scores used to rank the available remote experts **RE.** The quality metrics **37** can be displayed with the corresponding remote expert **RE,** and the remote experts can be ranked according to the highest quality metrics. The list **44** can also include "soon-to-be available remote experts **RE** based on the schedule availability data.

At an operation **110,** one of the remote experts **RE** listed on the list **44** is selected by the local operator **LO.** To do so, the local operator **LO** uses the at least one user input device **22'** and selects one of the listed remote experts **RE.** In some examples, the local operator **LO** can select the remote expert **RE** to open the drop down menu **46** to show the selected remote expert's set of experience metrics **48.** The local operator **LO** can also select the communication button **54** selectable by the remote expert **RE** or the local operator **LO** to establish the natural language communication pathway **19** via the communication link **14** between the two parties so that the screens of the medical imaging device controller **10** and the remote workstation **12** are shared. In some examples, the selected remote expert **RE** can decline the natural language communication pathway **19** (e.g., if the remote expert **RE** is busy, or feels that the problem to be addressed in the medical imaging examination does not suit his or her skills). In this case, the communication link can be established between another one of the listed remote experts **RE** (e.g., the next highest ranked remote expert). This process can continue until one of the remote experts **RE** accepts the natural language communication pathway **19.**

At an optional operation **112,** the database **41** can be updated based on the interactions between the selected remote expert **RE** and the local operator **LO.** In one example, if the selected remote expert **RE** declines to help the local operator **LO,** the database **41** can be updated to no longer recommend that remote expert for those types of problems, for that particular local operator **LO,** and so forth. In addition, data can be collected related to an effectiveness of assistance provided by the selected remote expert **RE** and the local operator **LO.** This can be done via a feedback form filled out by the local operator **LO** and/or the remote expert **RE.** In one example, one or more of the quality metrics **37** for the selected remote expert **RE** can be recalculated, and stored in the database **41** (along with updates related to the additions to the remote expert's experience through handling the matter with the local operator **LO**). In another example, an adaptive-learning process on the collected data to maximize the quality metrics 37 related to the effectiveness of the assistance, which can also be stored in the database **41.**

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims.

## Claims

1. A computer-implemented method executable by at least one electronic processor (**14s**), of connecting a local medical imaging device operator (**LO**) with a remote medical imaging expert (**RE**) during an imaging examination performed using a medical imaging device (**2**), the method comprising:
determining characteristics for available remote medical imaging experts who are available to assist the local operator;
matching one or more of the available remote medical imaging experts based on the determined characteristics for the remote medical imaging experts with characteristics of the imaging examination; and
providing a user interface (UI) (**28'**) to at least one display device (**24**') operable by the local operator, the UI displaying a list (**44**) of the matched available remote medical imaging experts and via which the local medical imaging device operator can select a matched available medical imaging expert from the displayed list,
wherein determining characteristics for remote medical imaging experts (**RE**) of a set of remote medical imaging experts available to assist the local operator (**LO**) includes:
performing a feature selection process on the characteristics of the remote medical imaging experts and the characteristics of the imaging examination.

2. The method of claim 1, wherein the method further includes:
ranking the available remote medical imaging experts (**RE**) based on a score (**37**) of the characteristics of the available remote medical imaging experts and the characteristics of the imaging examination.

3. The method of claim 2, wherein providing the UI displaying the list (**44**) of the matched available remote medical imaging experts (**RE**) includes:
providing the list as a ranked list of the available remote medical imaging experts according the scores (**37**).

4. The method of claim 2, wherein providing the UI (**28**') displaying the list (**44**) of the matched available remote medical imaging experts (**RE**) includes:
providing the list including the highest ranked available remote medical imaging experts according to the scores (**37**).

5. The method of either one of claims 3 and 4, wherein providing the UI (**28'**) displaying the list (**44**) of the matched available remote medical imaging experts (**RE**) includes:
listing the scores (**37**) of the listed available remote medical imaging experts on the UI.

6. The method of any one of claims 3-5, wherein the determining of the characteristics for available remote medical imaging experts (**RE**) includes determining a scheduling availability of the remote medical imaging experts; and the providing of the UI (**28'**) displaying the list (**44**) includes providing the list with the scheduling availability of the remote medical imaging experts.

7. The method of any one of claims 2-6, wherein ranking the available remote medical imaging experts (**RE**) includes:
applying a machine learning (ML) operation to the characteristics of the available remote medical imaging experts to rank the available remote medical imaging experts.

8. The method of any one of claims 1-7, wherein the characteristics of the available remote medical imaging experts (**RE**) include at least one of:
an experience level with an imaging modality of the imaging examination;
an experience level with an anatomy of a patient to be imaged during the imaging examination;
an experience level of working with the local operator (**LO**);
an experience level with a type of problem occurring in the imaging examination.

9. The method of any one of claims 1-8, wherein providing the UI (**28**') displaying a list (**44**) of the matched available remote medical imaging experts (**RE**) includes:
upon receiving a user input via at least one user input device (**22'**) indicative of a selection of one of the listed remote medical imaging experts, providing a drop-down menu (**46**, **52**) displaying information about the selected remote medical imaging expert.

10. The method of any one of claims 1-9, wherein providing the UI (**28**') displaying a list (**44**) of the matched available remote medical imaging experts (**RE**) includes:
upon receiving a user input via at least one user input device (**22'**) indicative of a selection of one of the listed remote medical imaging experts, establishing a two-way telephonic or video communication link (**14, 19**) between the selected remote medical imaging expert and the local operator (**LO**) and sharing a display of the medical imaging device (**2**) at a workstation (**12**) of the selected remote medical imaging expert.

11. The method of claim 10, wherein providing the UI (**28'**) displaying a list (**44**) of the matched available remote medical imaging experts (**RE**) includes:
upon receiving an indication from the selected remote medical imaging expert of a decline of the communication link, establishing a communication link (**14, 19**) between another one of the listed remote medical imaging experts and the local operator (**LO**).

12. The method of any one of claims 1-11, wherein the characteristics of the remote medical imaging experts (**RE**) are stored in a database, and the method further includes:
collecting data related to an effectiveness of assistance provided by the selected remote medical imaging expert and the local operator (**LO**);
calculating one or more quality metrics (**37**) related to the collected data; and
updating the information stored in the database for the selected remote medical imaging expert with the calculated one or more quality metrics.

13. An apparatus (**10**) configured to carry out a method as claimed in any one of the preceding claims, the apparatus comprising:
a communication link (**19**) operatively connected with an electronic network (**14**) for providing communication with a remote medical imaging expert (**RE**) of a set of remote medical imaging experts;
a database (**41**) storing determining characteristics for the remote medical imaging experts of the set of remote medical imaging experts, the characteristics including at least one of: an experience level with a modality of the imaging examination; an experience level with an anatomy of a patient to be imaged during the imaging examination; an experience level of working with the local operator; and an experience level with a type of problem occurring in the imaging examination; and
at least one electronic processor (**14s**) programmed to carry out the method of any one of the preceding claims.

14. The apparatus (**10**) of claim 13, wherein the at least one electronic processor (**14s**) is further programmed to:
upon receiving a user input via at least one user input device (**22, 22**') indicative of a selection of one of the listed remote medical imaging experts (**RE**), establishing a communication link (**14, 19**) between the selected remote medical imaging expert and the local operator (**LO**).

15. A computer-readable storage medium comprising instructions which, when executed by a computer, cause the computer to carry out the steps of the method of any one of claims 1-12.

## Patentansprüche

1. Computerimplementiertes Verfahren, das von mindestens einem elektronischen Prozessor (**14s**) ausgeführt werden kann, um einen lokalen Bediener einer medizinischen Bildgebungsvorrichtung (**LO**) mit einem Experten für telemedizinische Bildgebung (**RE**) während einer mit einer medizinischen Bildgebungsvorrichtung (**2**) durchgeführten bildgebenden Untersuchung zu verbinden, wobei das Verfahren Folgendes umfasst:
Bestimmen der Eigenschaften verfügbarer Experten für telemedizinische Bildgebung, die dem lokalen Bediener zur Unterstützung zur Verfügung stehen;
Zuordnen eines oder mehrerer verfügbarer Experten für telemedizinische Bildgebung basierend auf den bestimmten Eigenschaften dieser Experten für telemedizinische Bildgebung zu den Eigenschaften der bildgebenden Untersuchung; und
Bereitstellen einer Benutzerschnittstelle (UI) (**28**') für mindestens eine Anzeigevorrichtung (**24**'), die vom lokalen Bediener bedient werden kann, wobei die UI eine Liste (**44**) der zugeordneten verfügbaren Experten für telemedizinische Bildgebung anzeigt und über die der lokale Bediener der medizinischen Bildgebungsvorrichtung einen zugeordneten verfügbaren Experten für medizinische Bildgebung aus der angezeigten Liste auswählen kann,
wobei das Bestimmen der Eigenschaften von Experten für telemedizinische Bildgebung (**RE**) aus einer Gruppe von Experten für telemedizinische Bildgebung, die dem lokalen Bediener (**LO**) zur Unterstützung zur Verfügung stehen, Folgendes einschließt:
Durchführen eines Merkmalsauswahlprozesses auf den Eigenschaften der Experten für telemedizinische Bildgebung und den Eigenschaften der bildgebenden Untersuchung.

2. Verfahren nach Anspruch 1, wobei das Verfahren weiter Folgendes einschließt:
Rangieren der verfügbaren Experten für telemedizinische Bildgebung (**RE**) auf der Grundlage einer Punktzahl (**37**) der Eigenschaften der verfügbaren Experten für telemedizinische Bildgebung und der Eigenschaften der bildgebenden Untersuchung.

3. Verfahren nach Anspruch 2, wobei Bereitstellen der UI zur Anzeige der Liste (**44**) der zugeordneten verfügbaren Experten für telemedizinische Bildgebung (**RE**) Folgendes einschließt:
Bereitstellen der Liste als Rangliste der verfügbaren Experten für telemedizinische Bildgebung nach den Punktzahlen (**37**).

4. Verfahren nach Anspruch 2, wobei Bereitstellen der UI (**28**'), die die Liste (**44**) der zugeordneten verfügbaren Experten für telemedizinische Bildgebung (**RE**) anzeigt, Folgendes einschließt:
Bereitstellen der Liste einschließlich der höchstrangigen verfügbaren Experten für telemedizinische Bildgebung gemäß den Punktzahlen (**37**).

5. Verfahren nach einem der Ansprüche 3 und 4, wobei Bereitstellen der UI (**28**'), die die Liste (**44**) der zugeordneten verfügbaren Experten für telemedizinische Bildgebung (**RE**) anzeigt, Folgendes einschließt:
Auflisten der Punktzahlen (37) der aufgelisteten verfügbaren Experten für telemedizinische Bildgebung auf der Ul.

6. Verfahren nach einem der Ansprüche 3-5, wobei das Bestimmen der Eigenschaften für verfügbare Experten für telemedizinische Bildgebung (**RE**) Bestimmen der Terminverfügbarkeit der Experten für telemedizinische Bildgebung einschließt; und das Bereitstellen der UI (**28**'), die die Liste (**44**) anzeigt, Bereitstellen der Liste mit der Terminverfügbarkeit der Experten für telemedizinische Bildgebung einschließt.

7. Verfahren nach einem der Ansprüche 2-6, wobei Rangieren der verfügbaren Experten für telemedizinische Bildgebung (**RE**) Folgendes einschließt:
Anwendung eines Maschinlern- (ML-) Vorgangs auf die Eigenschaften der verfügbaren Experten für telemedizinische Bildgebung, um die verfügbaren Experten für telemedizinische Bildgebung zu rangieren.

8. Verfahren nach einem der Ansprüche 1-7, wobei die Eigenschaften der verfügbaren Experten für telemedizinische Bildgebung (**RE**) mindestens eines einschließen von:
einem Erfahrungsniveau mit einer Bildgebungsmodalität der bildgebenden Untersuchung;
einem Erfahrungsniveau mit einer Anatomie eines Patienten, die während der bildgebenden Untersuchung abgebildet werden soll;
einem Erfahrungsniveau in der Zusammenarbeit mit dem lokalen Bediener (**LO**);
einem Erfahrungsniveau mit einer Art von Problem, das bei der bildgebenden Untersuchung auftritt.

9. Verfahren nach einem der Ansprüche 1-8, wobei Bereitstellen der UI (**28**'), die eine Liste (**44**) der zugeordneten verfügbaren Experten für telemedizinische Bildgebung (**RE**) anzeigt, Folgendes einschließt:
nach Empfangen einer Benutzereingabe über mindestens eine Benutzereingabevorrichtung (**22**'), die die Auswahl eines der aufgelisteten Experten für telemedizinische Bildgebung angibt, Bereitstellen eines Dropdown-Menüs (**46, 52**), das Informationen über den ausgewählten Experten für telemedizinische Bildgebung anzeigt.

10. Verfahren nach einem der Ansprüche 1-9, wobei Bereitstellen der UI (**28**'), die eine Liste (**44**) der zugeordneten verfügbaren Experten für telemedizinische Bildgebung (**RE**) anzeigt, Folgendes einschließt:
nach Empfangen einer Benutzereingabe über mindestens eine Benutzereingabevorrichtung (**22**'), die die Auswahl eines der aufgelisteten Experten für telemedizinische Bildgebung angibt, Herstellen einer bidirektionalen Telefon- oder Video-Kommunikationsverbindung (**14, 19**) zwischen dem ausgewählten Experten für telemedizinische Bildgebung und dem lokalen Bediener (**LO**) und Teilen einer Anzeige der medizinischen Bildgebungsvorrichtung (**2**) an einem Arbeitsplatz (**12**) des ausgewählten Experten für telemedizinische Bildgebung.

11. Verfahren nach Anspruch 10, wobei Bereitstellen der UI (**28**') Anzeigen einer Liste (**44**) der zugeordneten verfügbaren Experten für telemedizinische Bildgebung (R**E**) Folgendes einschließt:
nach Empfangen einer Angabe des ausgewählten Experten für telemedizinische Bildgebung über ein Ablehnen der Kommunikationsverbindung, Herstellen einer Kommunikationsverbindung (**14, 19**) zwischen einem anderen der aufgelisteten Experten für telemedizinische Bildgebung und dem lokalen Bediener (**LO**).

12. Verfahren nach einem der Ansprüche 1-11, wobei die Eigenschaften der Experten für telemedizinische Bildgebung (**RE**) in einer Datenbank gespeichert sind, und das Verfahren weiter Folgendes einschließt:
Erhebung von Daten in Bezug auf eine Effektivität der durch den ausgewählten Experten für telemedizinische Bildgebung bereitgestellten Unterstützung und den lokalen Bediener (**LO**);
Berechnen einer oder mehrerer Qualitätsmetriken (**37**) in Bezug auf die erhobenen Daten; und
Aktualisieren der in der Datenbank gespeicherten Informationen über den ausgewählten Experten für telemedizinische Bildgebung anhand der berechneten einen oder mehreren Qualitätsmetriken.

13. Einrichtung (**10**), die zum Ausführen eines Verfahrens nach einem der vorstehenden Ansprüche konfiguriert ist, wobei die Einrichtung Folgendes umfasst:
eine Kommunikationsverbindung (**19**), die operativ mit einem elektronischen Netzwerk (**14**) verbunden ist, um eine Kommunikation mit einem Experten für telemedizinische Bildgebung (**RE**) aus einer Gruppe von Experten für telemedizinische Bildgebung bereitzustellen;
eine Datenbank (**41**), die bestimmende Eigenschaften für die Experten für telemedizinische Bildgebung der Gruppe der Experten für telemedizinische Bildgebung speichert, wobei die Eigenschaften mindestens eines einschließen von: einem Erfahrungsniveau mit einer Modalität der bildgebenden Untersuchung; einem Erfahrungsniveau mit der Anatomie eines Patienten, der während der bildgebenden Untersuchung abgebildet werden soll; einem Erfahrungsniveau in der Zusammenarbeit mit dem lokalen Bediener; und einem Erfahrungsniveau mit einer Art von Problem, das bei der bildgebenden Untersuchung auftritt; und
mindestens einen elektronischen Prozessor (**14s**), der so programmiert ist, dass er das Verfahren nach einem der vorstehenden Ansprüche ausführt.

14. Einrichtung (**10**) nach Anspruch 13, wobei der mindestens eine elektronische Prozessor (**14s**) weiter programmiert ist zum:
nach Empfangen einer Benutzereingabe über mindestens ein Benutzereingabevorrichtung (**22, 22'**), die die Auswahl eines der aufgelisteten Experten für telemedizinische Bildgebung (**RE**) angibt, Herstellen einer Kommunikationsverbindung (**14, 19**) zwischen dem ausgewählten Experten für telemedizinische Bildgebung und dem lokalen Bediener (**LO**).

15. Computerlesbares Speichermedium, das Anweisungen umfasst, die, wenn sie von einem Computer ausgeführt werden, den Computer veranlassen, die Schritte des Verfahrens nach einem der Ansprüche 1-12 auszuführen.

## Revendications

1. Procédé mis en œuvre par ordinateur pouvant être exécuté par au moins un processeur électronique (**14s**), permettant de connecter un opérateur local de dispositif d'imagerie médicale (**LO**) avec un expert en imagerie médicale distant (**RE**) pendant un examen d'imagerie réalisé à l'aide d'un dispositif d'imagerie médicale (**2**), le procédé comprenant :
la détermination de caractéristiques d'experts en imagerie médicale distants disponibles qui sont disponibles pour assister l'opérateur local ;
la mise en correspondance d' un ou de plusieurs experts en imagerie médicale distants disponibles sur la base des caractéristiques déterminées pour les experts en imagerie médicale distants avec des caractéristiques de l'examen d'imagerie ; et
la fourniture d'une interface utilisateur (IU) (**28**') à au moins un dispositif d'affichage (**24**') utilisable par l'opérateur local, l'IU affichant une liste (**44**) des experts en imagerie médicale distants disponibles mis en correspondance, et via laquelle l'opérateur de dispositif d'imagerie médicale local peut sélectionner un expert en imagerie médicale disponible correspondant dans la liste affichée,
dans lequel la détermination de caractéristiques d'experts en imagerie médicale distants (**RE**) d'un ensemble d'experts en imagerie médicale distants disponibles pour assister l'opérateur local (**LO**) inclut :
la réalisation d'un processus de sélection de caractéristiques sur les caractéristiques des experts en imagerie médicale distants et les caractéristiques de l'examen d'imagerie.

2. Procédé selon la revendication 1, dans lequel le procédé inclut en outre :
le classement des experts en imagerie médicale distants disponibles (**RE**) sur la base d'un score (**37**) des caractéristiques des experts en imagerie médicale distants disponibles et des caractéristiques de l'examen d'imagerie.

3. Procédé selon la revendication 2, dans lequel la fourniture de l'UI affichant la liste (**44**) des experts en imagerie médicale distants disponibles mis en correspondance (**RE**) inclut :
la fourniture de la liste sous la forme d'une liste classée des experts en imagerie médicale distants disponibles selon les scores (**37**).

4. Procédé selon la revendication 2, dans lequel la fourniture de l'UI (**28**') affichant la liste (**44**) des experts en imagerie médicale distants disponibles mis en correspondance (**RE**) inclut :
la fourniture de la liste incluant les experts en imagerie médicale distants les mieux classés disponibles selon les scores (**37**).

5. Procédé selon l'une ou l'autre des revendications 3 et 4, dans lequel la fourniture de l'UI (**28**') affichant la liste (**44**) des experts en imagerie médicale distants disponibles mis en correspondance (**RE**) inclut :
la liste des scores (**37**) des experts en imagerie médicale distants disponibles sur l'UI.

6. Procédé selon l'une quelconque des revendications 3-5, dans lequel la détermination des caractéristiques d'experts en imagerie médicale distants disponibles (**RE**) inclut la détermination d'une disponibilité de planification des experts en imagerie médicale distants ; et la fourniture de l'UI (**28**') affichant la liste (**44**) inclut la fourniture de la liste avec la disponibilité de planification des experts en imagerie médicale distants.

7. Procédé selon l'une quelconque des revendications 2-6, dans lequel le classement des experts en imagerie médicale distants disponibles (**RE**) inclut :
l'application d'une opération d'apprentissage automatique (ML) aux caractéristiques des experts en imagerie médicale distants disponibles pour classer les experts en imagerie médicale distants disponibles.

8. Procédé selon l' une quelconque des revendications 1-7, dans lequel les caractéristiques des experts en imagerie médicale distants disponibles (**RE**) incluent au moins un :
d'un niveau d'expérience avec une modalité d'imagerie de l'examen d'imagerie ;
d'un niveau d'expérience avec l'anatomie d'un patient à imager pendant l'examen d'imagerie ;
d'un niveau d'expérience de travail avec l'opérateur local (**LO**)) ;
d'un niveau d'expérience avec un type de problème survenant lors de l'examen d'imagerie.

9. Procédé selon l' une quelconque des revendications 1-8, dans lequel la fourniture de l'UI (**28**') affichant une liste (**44**) des experts en imagerie médicale distants disponibles mis en correspondance (**RE**) inclut :
lors de la réception d'une entrée utilisateur via au moins un dispositif d'entrée utilisateur (**22**') indiquant une sélection de l'un des experts en imagerie médicale distants listés, la fourniture d'un menu déroulant (**46, 52**) affichant des informations concernant l'expert en imagerie médicale distant sélectionné.

10. Procédé selon l' une quelconque des revendications 1-9, dans lequel la fourniture de l'UI (**28**') affichant une liste (**44**) des experts en imagerie médicale distants disponibles mis en correspondance (**RE**) inclut :
lors de la réception d'une entrée utilisateur via au moins un dispositif d'entrée utilisateur (**22**') indiquant la sélection de l'un des experts en imagerie médicale distants listés, l'établissement d'une liaison de communication téléphonique ou vidéo bidirectionnelle (**14,19**) entre l'expert en imagerie médicale distant sélectionné et l'opérateur local (**LO**) et le partage d'un affichage du dispositif d'imagerie médicale (**2**) sur un poste de travail (**12**) de l'expert en imagerie médicale distant sélectionné.

11. Procédé selon la revendication 10, dans lequel la fourniture de l'UI (**28**') affichant une liste (**44**) des experts en imagerie médicale distants disponibles mis en correspondance (**RE**) inclut :
dès réception d'une indication de l'expert en imagerie médicale distant sélectionné d'une baisse de la liaison de communication, l'établissement d'une liaison de communication (**14, 19**) entre un autre des experts en imagerie médicale distants listés et l'opérateur local (**LO**).

12. Procédé selon l'une quelconque des revendications 1-11, dans lequel les caractéristiques des experts en imagerie médicale distants (**RE**) sont stockées dans une base de données, et le procédé inclut en outre :
la collecte de données se rapportant à l'efficacité de l'assistance fournie par l'expert en imagerie médicale distant sélectionné et l'opérateur local (**LO**));
le calcul d'une ou de plusieurs mesures de qualité (**37**) se rapportant aux données collectées ; et
la mise à jour des informations stockées dans la base de données pour l'expert en imagerie médicale distant sélectionné avec les une ou plusieurs métriques de qualité calculées.

13. Appareil (**10**) configuré pour mettre en œuvre un procédé selon l'une quelconque des revendications précédentes, l'appareil comprenant :
une liaison de communication (**19**) connectée de manière fonctionnelle à un réseau électronique (**14**) pour assurer une communication avec un expert en imagerie médicale distant (**RE**) d'un ensemble d'experts en imagerie médicale distants ;
une base de données (**41**) stockant la détermination de caractéristiques des experts en imagerie médicale distants de l'ensemble d'experts en imagerie médicale distants, les caractéristiques incluant au moins un : d'un niveau d'expérience avec une modalité d'examen d'imagerie ; d'un niveau d'expérience avec l'anatomie d'un patient à imager pendant l'examen d'imagerie ; d'un niveau d'expérience de travail avec l'opérateur local ; et d'un niveau d'expérience avec un type de problème survenant lors de l'examen d'imagerie ; et
au moins un processeur électronique (**14s**) programmé pour mettre en œuvre le procédé selon l'une quelconque des revendications précédentes.

14. Appareil (**10**) selon la revendication 13, dans lequel le au moins un processeur électronique (14s) est en outre programmé pour :
lors de la réception d'une entrée utilisateur via au moins un dispositif d'entrée utilisateur (**22, 22**') indiquant la sélection de l'un des experts en imagerie médicale distants listés (**RE**), établir une liaison de communication (**14, 19**) entre l'expert en imagerie médicale distant sélectionné et l'opérateur local (**LO**).

15. Support de stockage lisible par ordinateur comprenant des instructions qui, lorsqu'elles sont exécutées par un ordinateur, amènent l'ordinateur à mettre en œuvre les étapes du procédé selon l'une quelconque des revendications 1-12.
